# EUROPEAN PATENT SPECIFICATION

(11) **EP 2 411 890 B1**
(45) Date of publication and mention of the grant of the patent: **12.12.2012**
(21) Application number: 10717668.7
(22) Date of filing: 18.03.2010
(51) Int. Cl.: A61B 5/00

(54) **INTERFACE TO A PLANAR SENSOR SYSTEM AND A CONTROL OF SAME**
SCHNITTSTELLE FÜR EIN PLANARES SENSORSYSTEM UND STEUERUNG DERSELBEN
INTERFACE POUR SYSTEME DE CAPTEUR PLANAIRE ET COMMANDE DE CELUI-CI

(30) Priority: 25.03.2009 FI 20095308
(43) Date of publication of application: 01.02.2012
(73) Proprietor: MariMils Oy, 01450 Vantaa (FI)
(72) Inventor: ROPPONEN, Antti, F-00400 Helsinki (FI)
(74) Representative: Laine, Terho Tapio
(86) International application number: PCT/FI2010/050209
(87) International publication number: WO 2010/109061

(56) References cited:
- WO-A1-2005/020171
- WO-A1-2006/003245
- WO-A1-2008/068387
- WO-A1-2008/131213
- US-A1- 2006 171 570
- US-A1- 2008 191 864
- US-B1- 6 515 586

## Description

### FIELD OF INVENTION

The present invention relates to a wireless interface used in a planar sensor system and to a method for controlling said interface.

### BACKGROUND OF THE INVENTION

Near-field imaging is nowadays used to monitor e.g. the movements of people in rooms. Planar sensors, for example, disposed in floor structures are used in systems based on it, by means of which planar sensors information about the position and state of a person being monitored are obtained by measuring the impedance changes caused by a conductive object, e.g. a person. This type of arrangement can be used e.g. to monitor elderly people, and more particularly their movements and vital functions, in nursing homes, etc.

Publication WO 2006/003245 presents one sensor construction, wherein the sensor is in web form and comprises sequential electrically conductive areas. The electrically conductive areas are typically metallic, and they can be formed on a substrate, e.g. as printed layers, laminated layers, etched layers, or films. The metal is typically aluminum or copper, as is disclosed in publication W02008068387A1. According to the publication in question, sensor webs can comprise two or more superimposed layers. In that case the first layer can comprise conductive areas and their leads, and the second layer e.g. RF loops and their leads. The sensor web comprises an output connection for connecting it with a connection cable to the control electronics for supplying measuring voltages and control signals via the connection in question.

For patient monitoring or corresponding this type of sensor system is connected to a monitoring system, from which an alarm can be received e.g. on a mobile phone about an emergency situation or about other exceptional circumstances.

In many professions, e.g. in hospitals, the personnel use a name badge, which comprises a picture of the person and information about him/her. Often these name badges also function as keycards. People often have with them a lot of other paraphernalia, such as keys, mobile phones and also wallets, so that they do not want to add to the paraphernalia they carry.

Document US 2008/0191864 A1 discloses an interactive training system capable of capturing and analysing the movement of a user in an interactive surface.

### SUMMARY OF THE INVENTION

The purpose of this invention is to achieve a new kind of interface for planar sensor systems

According to the invention, the planar sensor system comprises a small, independent, portable display device used as an interface, which display device is wirelessly in connection with a planar sensor below it, into which planar sensor an antenna system is fitted. According to the invention the information on the display of the display device can be changed according to its location by determining the position of the display device and by presenting the information intended be displayed in exactly that place on the display of the display device.

In the arrangement according to the invention the name badge can thus function as a versatile interface. The name badge thus functions as a display device, which can comprise a touch-sensitive display, and which can comprise a wireless data transfer connection operating on radio frequencies, such as e.g. ZigBee, so that it can communicate with another system of the building or similar.

The name badge according to the invention also comprises intelligence, e.g. a microcontroller, so that the display device and the radio can be controlled. This type of name badge can also be called a tag.

An ordinary LCD display, OLED display, etc., can function as the display device. Perhaps one of the displays best suited for this purpose is electronic paper, which has a high resolution, the display does not need backlighting and it does not consume energy except when the image on the display is changed. These types of displays are in use e.g. in electronic books. A touch-sensitive property can be connected to all these displays, either as a film on the surface of the display or as some type of capacitive sensor under the display. The information of the display can be shown always the right way up if an accelerometer to detect the direction of the gravitational field is added to the tag.

The characteristic features of the user interface according to the invention and of the method for controlling it are presented in detail in the independent claims, and the preferred embodiments of them in the other claims.

By means of the invention a solution can be very simply implemented, e.g. in retirement homes or corresponding, wherein an attending physician has a name badge on his/her chest, which then in a room provided with a sensor changes the information on the display in different positions as the physician moves in the room.

### SHORT DESCRIPTION OF THE DRAWINGS

In the following, the invention will be described in more detail by the aid of some embodiments with reference to the attached drawings, wherein
Figs. 1 - 3 present a simplified display device according to the invention, which operates in a floor-sensor system, and
Figs. 4 and 5 present a prior-art sensor foil construction provided with an antenna.

### DETAILED DESCRIPTION OF THE INVENTION

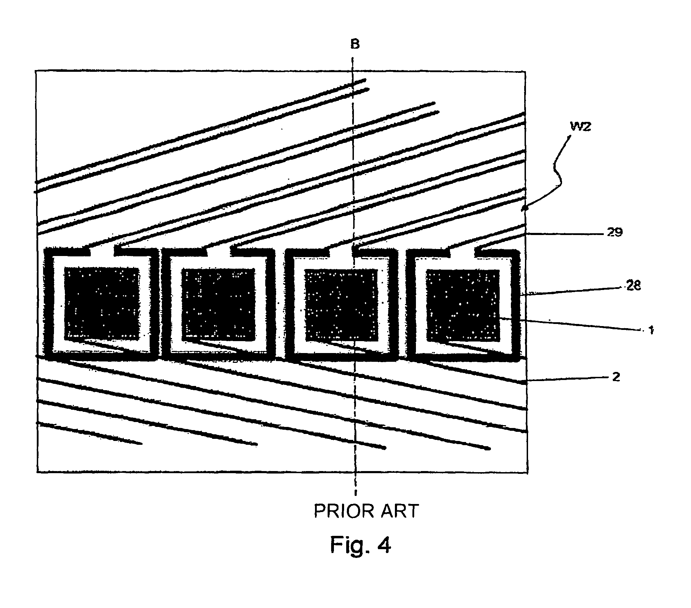

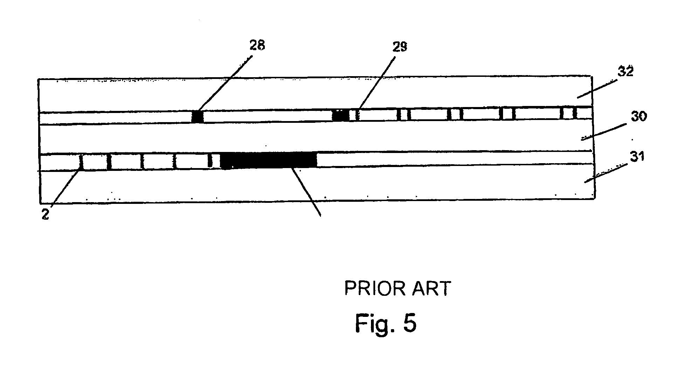

In this invention a name badge 11 functions as a versatile interface in a floor-sensor system 18 connected to a monitoring system e.g. in a hospital, which floor-sensor system comprises a floor sensor, e.g. according to Figs. 4 and 5, and a control unit 19 of said floor-sensor system.

Some type of display device 11, which comprises a display 12, e.g. a touch-sensitive display, and which can comprise an RF radio 13 so that it can communicate with another system of the building, thus functions as a name badge. The name badge comprises a microcontroller 14, with which the display device and the radio can be controlled. An ordinary LCD display, OLED display, or electronic paper can function as the display. The information of the display can be shown always the right way up when it comprises an accelerometer 15 to detect the direction of the gravitational field. In addition, it comprises a chargeable battery 17.

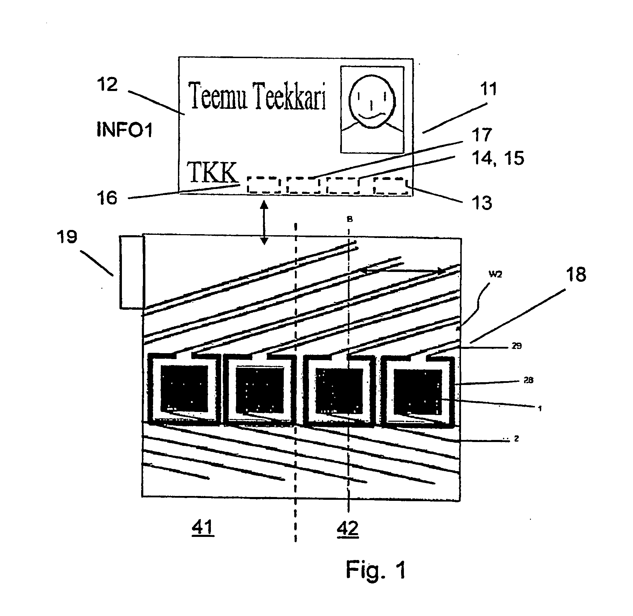

By default, the display functions as a normal name badge, which comprises name information according to the display INFO1 (Fig. 1). It can be carried normally visibly attached to a breast pocket, on some kind of belt or on a clip. The fastener must be of such a type that the name badge can be easily detached for handling or it must be at the end of some kind of stretchable belt, e.g. a yo-yo type belt. Thus the badge is easy to handle, when the name badge changes its operating mode.

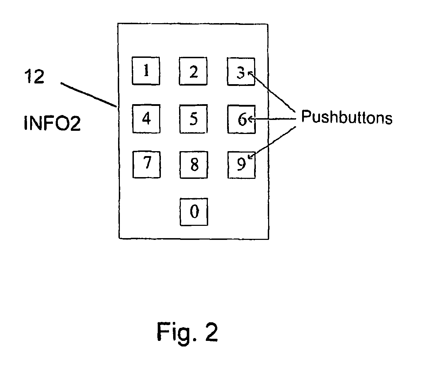

In addition, the name badge/tag comprises a short-range transmitter-receiver 16, which activates the tag when it is taken near a transmitter point, i.e. near one of the antennas 28 (Fig. 4), from which antenna the name badge receives by means of an excitation, which can be e.g. pulse-shaped, a signal about display information connected to exactly the zone 41, 42 in question. In this case e.g. when the tag is taken near a door, which forms one zone 41, which requires a numerical code, the display of the tag changes from a name badge into a numeric keypad by means of the touch-sensitive display INFO2 (Fig. 2). The code can thus be keyed in with the device and sent by ZigBee radio back to the system, which opens the door if the code is correct. The door can, of course, also open automatically if the tag is taken to the transmitter devices of the door. Short-range radios are used in many RFID systems, so the technology for this exists. According to the invention the antenna 28 sends an identification only when the display device has come to the location of the antenna in question, in which case power consumption is minimized.

Additionally, e.g. in hospitals a transmitter apparatus can be near the beds (the second zone 42, which display comprises information relating to exactly the patient in question), which transmitter apparatus sends patient data to the tag when the physician/nurse approaches the bed. The display can be scrolled, in which case an almost unlimited amount of information can be presented on the display, if so desired. Some displays also allow folding, so that the display can be partly in a roll inside the device and if necessary it can be pulled to become larger.

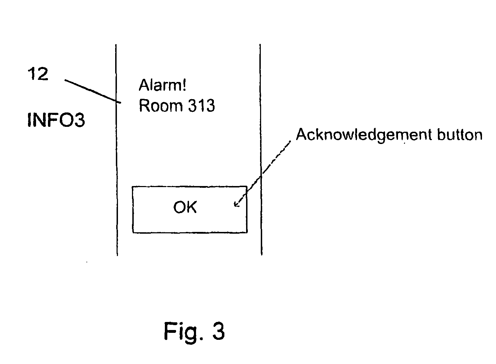

If the tag comprises a radio device 13 operating on a longer-range radio frequency, it can be used e.g. as a pager. For example, an alarm call is sent to a nurse if matters are going badly with some patient. An alarm can be raised automatically or by some person. In these situations e.g. an alarm and the position from which it originates are displayed on the display, and also some type of acknowledgement button with which it can be reported to the system that the message has been received (INF03, Fig. 3). In these types of cases it is good of the tag comprises a vibration or sound alarm, so that the nurse is able to react to alarms. Longer-range radio apparatuses are e.g. WLAN, Bluetooth or ZigBee. Video imaging or alternatively speech can also be sent via this type of radio contact, if the name badge comprises a microphone and a loudspeaker.

Fig. 4 presents a prior-art planar sensor foil construction known from WO 2008/068387 and applicable to the arrangement of the invention, comprising a sensor web W2, which can be used to monitor electrically conductive points, e.g. the movements and position in a room of a person, by means of capacitive detection. The sensor foil covers the whole room, and comprises webs W2, each of which comprises a number of adjacent, e.g. square-shaped conductive foil areas 1 typically 300 x 300 mm in size. They are connected to outside the foil by means of film-type connection leads 2, in addition to which the foil comprises a second layer, which comprises RF loops 28, which are arranged as a loop surrounding each square-shaped conductive area or a part of them, and the connection leads 29 of them. Owing to this type of closed loop, the excitation signal obtained from a control unit can be supplied to the name badge above it. Fig. 5 presents a cross-section of the sensor web (section B - B) of Fig. 4. The sensor web comprises two layers, a first layer 30 and a second layer 31, which are connected to each other. The first layer contains RF loops and their conductors 29. The second layer 31 contains electrically conductive areas 1 and their connection leads 2.

Detection of the position of a person in the area of the sensor foil functions e.g. so that a low-frequency, low-voltage carrier-wave signal is supplied to an individual element, i.e. to a conductive area (primary element), while the other elements are earthed to form a return path for the signal. Impedance forms in a conductive object, e.g. a person on the floor, from connection of the electrical field between the primary elements and the secondary elements, from the resistive properties of the conductive object, and from the inductance of all the conductors. The conductive object above the sensor thus affects the impedance and the current between the primary elements and the secondary elements. The position detection system measures the changes of impedance, on the basis of which the position of an object can be determined with a sensor.

It is obvious to the person skilled in the art that the different embodiments of the invention are not limited solely to the examples described above, but that they may be varied within the scope of the claims presented below. It is not needed to install an antenna that sends an excitation signal around every conductive area, but instead e.g. only in certain conductive areas of the sensor web. The display can be of two parts, such that some of the display is electronic paper and some of the display is a touch-sensitive display, which activates only e.g. near the door area.

## Claims

1. Planar sensor system, which planar sensor system comprises an electrically conductive planar sensor (18), which comprises a number of adjacent electrically conductive areas (1) and a control unit (19),
the planar sensor system comprises further a wireless interface (11),
the wireless interface is an independent display device, which comprises a display (12), a control unit (14), a power source, and also a short-range radio (16), and
which display can be controlled in the area of said planar sensor system dependence of the position **characterized in that**:
- the position of the display device is determined by means of the planar sensor,
- the information on the display in predefined positions is changed by forming a wireless connection between an antenna (28) arranged within the planar sensor and the display device by means of short-range radio, and by giving an excitation for displaying the information (INFO1 - INF03) predefined for the position in question on the display of the display device by means of the antenna.

2. Planar sensor system according to claim 1, **characterized in that** the area of the planar sensor, e.g. the floor of a room, is divided into zones (41, 42), and in the different zones information intended for the zone in question can be arranged on the display.

3. Planar sensor system according to claim 1, **characterized in that** the display is electronic paper.

4. Planar sensor system according to claim 1, **characterized in that** the sensor comprises adjacent, e.g. square-shaped conductive areas (1), and RF loops (28) that function as antennas, which are arranged as a loop surrounding at least a part of the conductive areas.

5. Planar sensor system according to claim 1, **characterized in that** it also comprises a longer-range radio apparatus that operates at a radio frequency, such as WLAN, Bluetooth or ZigBee.

6. Planar sensor system according to claim 1, **characterized in that** the display device comprises a pushbutton arrangement.

7. Planar sensor system according to claim 1, **characterized in that**:
the display device comprises a microphone and/or a loudspeaker.

8. Planar sensor system according to claim 1, **characterized in that** the display is a touch-sensitive display, in which case it can be changed e.g. from a name badge into a numeric keypad (INF02).

9. Planar sensor system according to claim 1, **characterized in that** the display is of multiple parts, such that some of the display is electronic paper and some of the display is a touch-sensitive display.

10. Method for controlling a planar sensor system, which planar sensor system comprises an electrically conductive planar sensor, which comprises a number of adjacent electrically conductive areas (1) and a control unit (19),
the planar sensor system comprises further a wireless interface (11), and
the interface is an independent display device, which comprises a display (12), a control unit (14), a power source, and also a short-range radio (16),
which display can be controlled in the area of said planar sensor system in dependency of the position charaterized by
- determining the position of the display device by means of the planar sensor,
- changing the information on the display in predefined positions by forming a wireless connection between an antenna (28) arranged within the planar sensor and the display device by means of short-range radio, and by giving an excitation for displaying the information (INFO1 - INFO3) predefined for the position in question on the display of the display device by means of the antenna.

11. Method according to claim 10, **characterized by** dividing the area of the planar sensor, into zones (41, 42), and by changing the different zones information intended for the zone in question on the display.

12. Method according to claim 10, **characterized by** providing an electronic paper for the display.

13. Method according to claim 10, **characterized by** providing the sensor with adjacent, conductive areas (1), and RF loops (28) that function as antennas, which are arranged as a loop surrounding at least a part of the conductive areas.

## Patentansprüche

1. Planares Sensorsystem, wobei das planare Sensorsystem einen elektrisch leitfähigen planaren Sensor (18) aufweist, der eine Anzahl von benachbarten elektrisch leitfähigen Bereichen (1) und eine Steuereinheit (19) aufweist,
wobei das planare Sensorsystem ferner eine drahtlose Schnittstelle (11) aufweist,
wobei die drahtlose Schnittstelle eine unabhängige Anzeigevorrichtung ist, die eine Anzeige (12), eine Steuereinheit (14), eine Energiequelle und auch eine Funkeinrichtung (16) mit geringer Reichweite aufweist, und
wobei die Anzeige in dem Bereich des planaren Sensorsystems in Abhängigkeit von der Position gesteuert werden kann, **dadurch gekennzeichnet, dass**:
- die Position der Anzeigevorrichtung mittels des planaren Sensorsystems bestimmt wird,
- die Information auf der Anzeige in vordefinierten Positionen verändert wird, indem eine drahtlose Verbindung zwischen einer Antenne (28), die innerhalb des planaren Sensorsystems angeordnet ist, und der Anzeigevorrichtung mittels der Funkeinrichtung mit geringer Reichweite geschaffen wird und indem eine Anregung zum Anzeigen der für die infrage kommende Position vordefinierten Information (INF01 - INFO3) auf der Anzeige der Anzeigevorrichtung mittels der Antenne gegeben wird.

2. Planares Sensorsystem nach Anspruch 1, **dadurch gekennzeichnet, dass** der Bereich des planaren Sensors, z.B. der Boden eines Raums, in Zonen (41, 42) unterteilt ist und in den verschiedenen Zonen für die infrage kommende Zone vorgesehene Information auf der Anzeige angeordnet werden kann.

3. Planares Sensorsystem nach Anspruch 1, **dadurch gekennzeichnet, dass** die Anzeige ein elektronisches Papier ist.

4. Planares Sensorsystem nach Anspruch 1, **dadurch gekennzeichnet, dass** der Sensor benachbarte, z.B. quadratische leitfähige Bereiche (1) und HF-Schleifen (28) aufweist, die als Antennen funktionieren, die als eine wenigstens einen Teil der leitfähigen Bereiche umgebende Schleife angeordnet sind.

5. Planares Sensorsystem nach Anspruch 1, **dadurch gekennzeichnet, dass** es ferner eine Funkvorrichtung mit größerer Reichweite, die mit einer Funkfrequenz arbeitet, wie beispielsweise WLAN, Bluetooth oder ZigBee, aufweist.

6. Planares Sensorsystem nach Anspruch 1, **dadurch gekennzeichnet, dass** die Anzeigevorrichtung eine Tastenanordnung aufweist.

7. Planares Sensorsystem nach Anspruch 1, **dadurch gekennzeichnet, dass**
die Anzeigevorrichtung ein Mikrophon und/oder einen Lautsprecher aufweist.

8. Planares Sensorsystem nach Anspruch 1, **dadurch gekennzeichnet, dass** die Anzeige eine berührungsempfindliche Anzeige ist, wobei sie in diesem Fall z.B. von einem Namensschild zu einem numerischen Tastenfeld (INF02) verändert werden kann.

9. Planares Sensorsystem nach Anspruch 1, **dadurch gekennzeichnet, dass** die Anzeige aus mehreren Teilen besteht, so dass ein Teil der Anzeige elektronisches Papier ist und ein Teil der Anzeige eine berührungsempfindliche Anzeige ist.

10. Verfahren zum Steuern eines planaren Sensorsystems, wobei das planare Sensorsystem einen elektrisch leitfähigen planaren Sensor aufweist, der eine Anzahl benachbarter elektrisch leitfähiger Bereiche (11) und eine Steuereinheit (19) aufweist,
wobei das planare Sensorsystem ferner eine drahtlose Schnittstelle (11) aufweist und
die Schnittstelle eine unabhängige Anzeigevorrichtung ist, die eine Anzeige (12), eine Steuereinheit (14), eine Energiequelle und auch eine Funkeinrichtung (16) mit geringer Reichweite aufweist,
wobei die Anzeige in dem Bereich des planaren Sensorsystems in Abhängigkeit von der Position gesteuert werden kann, **gekennzeichnet durch**
- Bestimmen der Position der Anzeigevorrichtung mittels des planaren Sensors,
- Verändern der Information auf der Anzeige in vordefinierten Positionen **durch** Herstellen einer drahtlosen Verbindung zwischen einer Antenne (28), die innerhalb des planaren Sensors angeordnet ist, und der Anzeigevorrichtung mittels der Funkeinrichtung mit geringer Reichweite und **durch** Abgabe einer Anregung zum Anzeigen der für die infrage kommende Position vordefinierten Information (INF01 - INFO3) auf der Anzeige der Anzeigevorrichtung mittels der Antenne.

11. Verfahren nach Anspruch 10, **gekennzeichnet durch** Unterteilen des Bereichs des planaren Sensors in Zonen (41, 42) und **durch** Verändern der unterschiedlichen Zoneninformation, die für die infrage kommende Zone vorgesehen ist, auf der Anzeige.

12. Verfahren nach Anspruch 10, **gekennzeichnet durch** Bereitstellen eines elektronischen Papiers für die Anzeige.

13. Verfahren nach Anspruch 10, **gekennzeichnet durch** Versehen des Sensors mit benachbarten leitfähigen Bereichen (1) und HF-Schleifen (28), die als Antennen funktionieren, die als eine wenigstens einen Teil der leitfähigen Bereiche umgebende Schleife angeordnet sind.

## Revendications

1. Système de capteur plan, lequel système de capteur plan comprend un capteur plan (18) électriquement conducteur, qui comprend un certain nombre de surfaces (1) électriquement conductrices adjacentes, et une unité de commande (19),
le système de capteur plan comprenant en outre une interface sans fil (11),
l'interface sans fil étant un dispositif d'affichage indépendant, qui comprend un afficheur (12), une unité de commande (14), une source d'alimentation, et également une radio à courte portée (16), et
lequel afficheur peut être commandé dans la surface dudit système de capteur plan en fonction de la position, **caractérisé en ce que** :
- la position du dispositif d'affichage est déterminée au moyen du capteur plan,
- les informations sur l'afficheur à des positions prédéfinies sont modifiées en formant une connexion sans fil entre une antenne (28) agencée dans le capteur plan et le dispositif d'affichage au moyen d'une radio à courte portée, et en appliquant une excitation pour afficher les informations (INFO1 à INF03) prédéfinies pour la position en question sur l'afficheur du dispositif d'affichage au moyen de l'antenne.

2. Système de capteur plan selon la revendication 1, **caractérisé en ce que** la surface du capteur plan, par exemple le sol d'une salle, est divisée en zones (41, 42), et, dans les différentes zones, des informations destinées à la zone en question peuvent être agencées sur l'afficheur.

3. Système de capteur plan selon la revendication 1, **caractérisé en ce que** l'afficheur consiste en du papier électronique.

4. Système de capteur plan selon la revendication 1, **caractérisé en ce que** le capteur comprend des surfaces (1) conductrices par exemple de forme carrée adjacentes, et des boucles RF (28) qui fonctionnent en tant qu'antennes, qui sont agencées en tant que boucle entourant au moins une partie des surfaces conductrices.

5. Système de capteur plan selon la revendication 1, **caractérisé en ce qu'**il comprend également un appareil radio à plus longue portée qui fonctionne à une fréquence radio, tel que WLAN, Bluetooth ou ZigBee.

6. Système de capteur plan selon la revendication 1, **caractérisé en ce que** le dispositif d'affichage comprend un agencement de boutons-poussoirs.

7. Système de capteur plan selon la revendication 1, **caractérisé en ce que** :
le dispositif d'affichage comprend un microphone et/ou un haut-parleur.

8. Système de capteur plan selon la revendication 1, **caractérisé en ce que** l'afficheur est un afficheur tactile, auquel cas il peut être changé, par exemple, d'un insigne d'identité en un clavier numérique (INF02).

9. Système de capteur plan selon la revendication 1, **caractérisé en ce que** l'afficheur comporte de multiples parties, de sorte qu'une partie de l'afficheur consiste en du papier électronique et une partie de l'afficheur consiste en un afficheur tactile.

10. Procédé de commande d'un système de capteur plan, lequel système de capteur plan comprend un capteur plan électriquement conducteur, qui comprend un certain nombre de surfaces (1) électriquement conductrices adjacentes, et une unité de commande (19),
le système de capteur plan comprenant en outre une interface sans fil (11), et
l'interface sans fil étant un dispositif d'affichage indépendant, qui comprend un afficheur (12), une unité de commande (14), une source d'alimentation, et également une radio à courte portée (16), et
lequel afficheur peut être commandé dans la surface dudit système de capteur plan en fonction de la position, **caractérisé par** :
- la détermination de la position du dispositif d'affichage au moyen du capteur plan,
- la modification des informations sur l'afficheur à des positions prédéfinies en formant une connexion sans fil entre une antenne (28) agencée dans le capteur plan et le dispositif d'affichage au moyen d'une radio à courte portée, et en appliquant une excitation pour afficher les informations (INFO1 à INF03) prédéfinies pour la position en question sur l'afficheur du dispositif d'affichage au moyen de l'antenne.

11. Procédé selon la revendication 10, **caractérisé par** la division de la surface du capteur plan en zones (41, 42), et par le changement, dans les différentes zones, des informations destinées à la zone en question sur l'afficheur.

12. Procédé selon la revendication 10, **caractérisé par** la fourniture d'un papier électronique pour l'afficheur.

13. Procédé selon la revendication 10, **caractérisé en ce que** le capteur est pourvu de surfaces (1) conductrices adjacentes, et de boucles RF (28) qui fonctionnent en tant qu'antennes, qui sont agencées en tant que boucle entourant au moins une partie des surfaces conductrices.
